# EUROPEAN PATENT APPLICATION

(11) **EP 1 616 851 A1**
(43) Date of publication of application: **18.01.2006**
(21) Application number: 04103384.6
(22) Date of filing: 15.07.2004
(51) Int. Cl.: C07C 51/41, C07C 59/08

(54) **Method for the preparation of a stable lactate metal salt in powder form and stable metal lactate salt**

(71) Applicant: PURAC Biochem BV, 4206 AC Gorinchem (NL)
(72) Inventor: Geerse, Kees, Bert, 3011 KP, Rotterdam (NL); Roozen, Lambertus, Hendricus, Elisabeth, 5126 CB, Gilze (NL); Jansen, Peter, Paul, 5342 AM, Oss (NL)
(74) Representative: Beetz, Tom

(57) **Abstract**

The invention relates to a method for the preparation of a stable lactate metal salt in powder form, the product of said preparation, functional pre-mixes for foodstuff comprising said stable lactate metal salt powder, and foodstuffs comprising said stable lactate metal salt powder.

In the method according to the invention, a concentrate that contains lactate metal salt is processed, with cooling, in a mixer/extruder to form a powder of the lactate metal salt and subsequently the powder of the lactate metal salt is partially encapsulated by means of an encapsulating agent. According to the invention the alkali metal lactates in powder form are storage stable for at least one year.

## Description

The invention relates to the preparation of a stable lactate metal salt in powder form, in particular a stable alkali metal lactate in powder form, the product of said preparation, functional pre-mixes for foodstuff comprising said stable lactate metal salt powder, foodstuffs and cosmetics comprising said stable lactate metal salt powder.

Metal lactate salts, more particularly sodium lactate is, inter alia, an important constituent in various flavouring mixtures. For the preparation of a flavouring mixture in powder form it is important to be able to use sodium lactate in powder form. The stability during storage and of, in particular, open packs are important criteria here. For good processing of lactate metal salt in powder form in such flavouring mixtures the stability of the lactate metal salt in powder form in open packs, is preferably at least 24 hours. During storage the lactate metal salt powder should be free from caking for at least one year.

Also for the application of lactate metal salts in other fields such as cosmetics and detergents it is often important to have a product in powder form which does not cake and maintains its free flowing properties even upon storage.

Sodium lactate in powder form is currently produced by crystallisation of a concentrated sodium lactate solution in absolute ethanol. Because of its highly hygroscopic properties, this powder has limited stability; it absorbs moisture very rapidly and in doing so forms a viscous fluid (as a rule within one hour and often after only 15 minutes).

Netherlands Patent Application 7106959 discloses a method for the preparation of sodium lactate in powder form with which an aqueous sodium lactate solution is spray-dried in a spray tower. With this method a glassy product may be formed on the wall of the spray tower. This can largely be prevented by spray-drying a cooking salt solution first and then spray-drying the sodium lactate solution. This product however lacks the desired stability. The starting point for the present invention is a different method for removing water wherein lactate metal salt is made in powder form in a less-energy costly way. Furthermore, the powder obtained with said method was found to have the desired stability against caking. The invention provides a solution to the stability problem described above and relates to a method for the preparation of a stable lactate metal salt in powder form, wherein:
a) a concentrate that contains lactate metal salt is processed, with cooling, in a mixer/extruder to form a powder of the lactate metal salt, and
b) subsequently the powder of the lactate metal salt is partially encapsulated by means of an encapsulating agent to form a partially encapsulated lactate metal salt powder.

The encapsulation of food ingredients is known. For instance, lactic acid being widely used in the food industry such as in the dairy industry, the meat industry, the bakery industry and the confectionery industry, is also often reported as being used in a coated form in a food composition. See for instance EP 527,570, US 4,262,027, and US 4,576,825. As lactic acid is normally in the liquid from, it is necessary to combine it with a solid substrate in order to obtain a solid composition. In EP 527,570 it is combined with glucose, in US 4,497,845 with a solid carrier, in US 4,511,584 with micro-cellulose or calcium lactate, and in US 4,511,592, US 4,772,477, and US 6,153,236 the lactic acid is platted on calcium lactate. In US 4,576,825, liquid lactic acid is encapsulated using a co-axial-extrusion method. The encapsulation of lactate metal salts is not so obvious, because in most applications an encapsulation would hamper its functionality. For instance, when using sodium lactate in meat applications it is necessary that it solves in the meat immediately upon addition in order to have its antimicrobial and color preserving properties. The same goes for applications in other foodstuffs and cosmetics. We have found that with partial encapsulation the lactate metal salts have improved storage stability, while their functionality has not been hampered.

Thus, for the present invention it is essential that the lactate metal salt is only partially encapsulated, because otherwise the lactate metal salt' s functionality when applied is hampered. The encapsulation should be sufficient to prevent caking during storage. It was found that in general this partial encapsulation is obtained when using an encapsulating agent in an amount varying from 1 to 15% by weight of the total partially encapsulated lactate metal salt powder. Preferably about 5 to 95% of the surface of the lactate metal salt powder is encapsulated, more preferably, 10 to 50% and most preferably 10 to 40% of the surface of the lactate metal salt powder. The preferred amount of encapsulating agent varies from 4 to 8% by weight of the total partially encapsulated lactate metal salt powder. With these amounts the storage stability is ensured while the texture of the powder and its taste in foodstuffs is not adversely affected.

Suitable encapsulating agents may be chosen from hydrogenated oil, fat, wax, carbohydrates such as anti-oxidants and sugars, proteins, polymers, or mixtures thereof.

The mixing/extruding with cooling of a sodium lactate solution to form sodium lactate powder has been described in WO 03/031358. In this patent application however, the (partial) encapsulation is not described.

According to the invention, the lactate metal salt is preferably an alkali metal salt such as lithium lactate, sodium lactate or potassium lactate and in particular sodium lactate or an earth alkali metal lactate salt such as calcium lactate, magnesium lactate. Also zinc lactate may be suitably used in the method according to the present invention. According to the invention the alkali metal lactates in powder form are stable for at least one year in closed packs, while the stability in open packs is at least 24 hours.

The starting material used for the preparation of the concentrate is an aqueous solution or suspension of the lactate metal salt. As the lactate metal salt is commonly obtained by fermentation of carbohydrates, this 50-70% (m/m), preferably 55-65% (m/m), aqueous solution is preferably first treated with active charcoal before the solution is concentrated to 60-100% (m/m), preferably 80-100% (m/m), most preferably 90-100% (m/m).

According to the invention, the processing in an extruder/mixer is preferably carried out at a starting temperature of 110° C to 170° C, preferably 130° C to 165° C. The cooling ranges from 10° C to 100° C, preferably 20° C to 90° C. Suitable extrude/mixers are Haake Rheomix 600 Models ®, Hobart mixers, Werner& Pfliederer Models®, APV- Baker mixer/extruders, Simon Freres MXT models or any other comparable extruder/mixers known in the art.

If it is desirable or necessary to shorten the length of the mixer/extruder, the concentrate can first be cooled in a heat extractor column as a pre-treatment. In this case the concentrate is cooled, under the influence of gravity, in countercurrent with air/nitrogen in the heat extractor column, the concentrate being cooled by 20°- 50° C with respect to the starting temperature.

The lactate metal salt powder is preferably ground, preferably in a conical flourmill or a hammer mill, to the desired particle size before step (b). This particle size is preferably less than 800 µm and in particular is 200 to 800 µm.

Step (b) of the method according to the above-described embodiment is preferably carried out by adding the encapsulating agent and the lactate metal salt powder to a mixer, for example a Hobart mixer, a Turbula Nauta or Forberg mixer or its equivalents on industrial scale, and mixing at the desired temperature. In order to ensure that a free flowing powder is obtained it is preferred that (part) of step (b) is conducted under agitation.

The temperature desired depends on the type of encapsulating agent used. When melt coating is desired and, for instance, fat is used, the temperature should be high enough to ensure melting of the fat. Preferably the temperature of the powder to be encapsulated is increased to slightly above the melting temperature of the encapsulating agent.

A suitable encapsulation procedure comprises the addition of the encapsulating agent, increasing the temperature of the powder of the lactate metal salt to a temperature slightly, i.e. 2- 15° C, above the melting temperature of the encapsulating agent, the powder of the lactate metal salt is encapsulated, and cooling the resulting partially encapsulated lactate metal salt powder to room temperature under agitation.

In order to improve the stability of the lactate metal salt powder even more, the lactate metal salt may be combined with a carrier. Said combining with a carrier may be conducted prior to being processed with cooling in the mixer/extruder to directly form a powder comprising the alkali metal lactate. With this method the concentrate used, as a starting material may be relatively low concentrated: down to 60% (m/m). The lactate metal salt may also be combined with a carrier after processing the concentrate with an extruder/mixer, with cooling, i.e. prior to, during or after the encapsulation step. It was found that with this method a higher concentration of lactate metal salt in the powder may be obtained compared with the powder obtained with combined extrusion. In this method the ratio of lactate metal salt powder: carrier is at least 99 : 1 to 50 : 50 and preferably 80 : 20 based on the weight of the lactate metal salt comprising powder.

The carrier that is used herein is preferably a flour, a starch, a silicate or an alkaline earth metal lactate. The flour is preferably rice flour. The starch is preferably cornstarch, wheat starch or pea starch. The silicates are preferably food-grade silicas such as Sipernat ®22S and 50S, ex Degussa and Zeothix 265. The alkaline earth metal lactate is preferably calcium lactate. With this method the ratio of lactate metal salt: carrier varies from 50 : 50 to 10 : 90, and preferably 50 : 50 to 40 : 60 based on the weight of the lactate metal salt comprising powder.

The stability of the stable lactate metal salt in powder form can be even further increased by adding a suitable emulsifier, for example sodium stearyl lactate or lecithin, either during the processing in the extruder/mixture or prior to or during step (b). The stability and the product characteristics of the stable lactate metal salt in powder form can be adjusted with the aid of these additives, the requisite stability duration of at least 24 hours always being met.

The partially encapsulated lactate metal salt powder obtained with the methods according to the invention have a storage stability of at least one year which renders it novel and are therefore, also subject of this invention. Owing to its stability, the lactate metal salt powder according to the invention appears highly suitable for use in foodstuffs and even for functional pre-mixes for foodstuffs. Normally the pre-mix may comprise 20 to 80 wt.% lactate metal salt according to the invention. The partially encapsulated lactate metal salt (either in functional pre-mixes or not) according to the invention may advantageously be used in foodstuffs such as beverages, soups, sauces, meat, poultry and fish, including both fresh (e.g., cold cuts, ground meat, and marinated fresh meat) as cooked meat (e.g., emulsified and whole muscle). Additional ingredients for functional pre-mixes comprise spices, preservatives colourings and flavourings etcetera. Said pre-mix may be prepared by either combining the stable lactate metal salt-containing powder with the other ingredients or the other ingredients may be added to the lactate metal salt at any stage during preparation of partially encapsulated lactate metal salt powder. Irrespective of the preparation method used, the lactate metal salt powder according to the invention, before being used in various applications, is preferably ground to a particle size of less than 800 micrometers, more preferably to a particle size between 200-800 micrometers. The invention is also directed to foodstuffs and functional pre-mixes comprising the stable lactate metal salt powder according to the invention.

The invention is further elucidated by the examples, which are to be construed as illustrative only and not as being limitative.

### EXAMPLES

### General preparation partially encapsulated lactate metal salt powder

An aqueous solution of metal lactate that contained 60-65% (m/m) sodium lactate was evaporated, either under atmospheric pressure or under reduced pressure, to give a concentrate that contained 90% (m/m) metal lactate. In additional experiments the aqueous solutions that contained 60-65% (m/m) metal lactate were combined with an encapsulating agent and optionally a carrier. The encapsulating agent was hydrogenated palm oil. The carrier used was silica. The concentrate and optionally the carrier were processed in a Haake Rheomix 600 model mixer/extruder to give a powder comprising about 42% (m/m) metal lactate. The mixing times and processing temperatures were, respectively, 5 to 30 minutes and 90° C to 130° C. Prior to encapsulation the lactate metal salt powder was milled to obtain particles of between 200 and 800 micrometers. The encapsulation was done is a mixer. The temperature of the lactate metal salt powder was first increased to a temperature of 70-80° C under air blowing. Subsequently the temperature was decreased to about 45° C and the encapsulating agent was added, The mixture was mixed for about 10 minutes and the temperature was increased to about 60° C under agitation. The resulting partially encapsulated powder was cooled to room temperature under agitation. When the temperature of the powder is lower than 45° C optionally a carrier was added.

### Example 1

### Stability tests of partially encapsulated sodium lactate powder

Spice blends (50/50) were prepared with partially encapsulated sodium lactate powder (96% sodium lactate, 4.75% palm oil, 0.25 silica) and a frankfurter spice mix having the composition as described in TABLE 1. As a comparison also spice blends (50/50) were made with un-encapsulated sodium lactate powder (96% sodium lactate, 4% silica) and the same frankfurter spice blend.

**TABLE I Ingredients frankfurter spice blend**

| Ingredients | % |
|---|---|
| Dextrose | 33.33 |
| Maltodextrine | 33.33 |
| Phosphate | 16.67 |
| Sodium ascorbate | 1.67 |
| Sodium glucomate | 1.67 |
| Pepper (white) | 6.67 |
| Cilantro | 3.33 |
| Mace | 3.33 |

The stability of the spice blend was tested by filling closed bottles with the spice blend and storage at 20 and 30° C. The moisture content of the samples was 4.7%.
Upon storage at 20° C, the spice blend with partially encapsulated sodium lactate powder remained free flowing for at least 117 days, while the spice blend with the un-encapsulated sodium lactate powder showed free-flowing properties with agglomeration after 25 days and became slightly caked after 117 days.
Upon storage at 30° C, the spice blend with partially encapsulated sodium lactate powder became caked after 76 days, while the spice blend with the un-encapsulated sodium lactate powder became caked after 25 days.

### Example 2

### Optimal encapsulation

Several partially encapsulated sodium lactate powders were prepared according to the general preparation method described above, having different amounts of encapsulating agent, i.e. 10.7, 8.7, 6.7, 4.7, 2.7 and 0% respectively. The sample with 0% encapsulating agent contained 4% silica while the other samples contained no silica. Blends were prepared with the frankfurter spice mix of Example 1. The spice blends were stored in a closed bottle at 20 and 30° C, respectively, with a moisture content of 3.1%. Upon storage at 20° C, all samples stayed free flowing for at least three months, except for the sample with no encapsulating agent: this sample caked after only one day.

Upon storage at 30° C, the sample with 0% encapsulating agent was caked at day 1. The sample with 2.7% encapsulating agent started caking after one month. The samples with more encapsulating agent remained free flowing for at least two months.

Further the spice blends were stored in closed bottles at 30° C with moisture content of 5.4. At this moisture content, the samples of 10.7% were sticky and thus, did not have the right texture. Accordingly, samples with 4 to 8 wt.% encapsulating agent were found the have the right texture and storage stability.

### Example 3

### Stability in open pots

Spice blends with different ratios (20:80 , 67:33, 50:50, 0:100 w/w) were prepared with partially encapsulated sodium lactate powder (96% sodium lactate, 4.75% palm oil, 0.25 silicate) and a frankfurter spice mix having the composition as described in TABLE 1. As a comparison also spice blends with the same ratios were made with un-encapsulated sodium lactate powder (96% sodium lactate, 4% silica) and the same frankfurter spice blend.

The resulting mixes were stored in open pots at 20° C and 60% RH. The samples were checked on their stabilities by visual observation.
The results are compiled in TABLE II.

**TABLE II stability in open pots at 20° C and 60% RH**

| **Samples** | **0 h** | **2 h** | **4h** | **6h** | **8h** | **24h** | **48h** | **56h** | **5 days** |
|---|---|---|---|---|---|---|---|---|---|
| 80:20 | F | F | F-a | F-A | SC | SC | C | C | C |
| spice blend: encapsulated | | | | | | | | | |
| 80:20 | F | F-A | F-A | C | C | C | C | C | C |
| spice blend:un-encapsulated | | | | | | | | | |
| 67:33 | F | F | F-a | F-A | F-A | F-A | C | C | C |
| spice blend:encapsulated | | | | | | | | | |
| 67:33 | F | F-A | F-A/SC | C | C | C | C | C | C |
| spice blend:un-encapsulated | | | | | | | | | |
| 50:50 | F | F | F-a | F-a | F-a | F-A | SC | C | C |
| spice blend:encapsulated | | | | | | | | | |
| 50:50 | F | F | F-A | C | C | C | C | C | C |
| spice blend:un-encapsulated | | | | | | | | | |
| 100% encapsulated | F | F | F | F | F | F | F-A | F-A | SC |
| 100% un-encapsulated | F | F-A | F-A/SC | SC | C | C | C | C | C |

| | | | | | | | | | |
|---|---|---|---|---|---|---|---|---|---|
| F= free flowing, F-a= Free flowing with slight agglomeration, F-A=Free flowing with agglomeration, SC=slightly caking, C= caking | | | | | | | | | |

## Claims

1. Method for the preparation of a stable lactate metal salt in powder form, wherein:
a) a concentrate that contains lactate metal salt is processed, under cooling, in a mixer/extruder to form a powder of the lactate metal salt, and
b) subsequently the powder of the lactate metal salt is partially encapsulated by means of an encapsulating agent to form a partially encapsulated lactate metal salt powder.

2. Method according to claim 1 wherein step (b) is conducted under agitation.

3. Method according to claim 1 or 2 wherein the encapsulation is done by melt coating.

4. Method according to claim 3, wherein during step (b) the encapsulating agent is added, the temperature of the powder of the lactate metal salt is increased to a temperature slightly above the melting temperature of the encapsulating agent, the powder of the lactate metal salt is encapsulated, and the resulting partially encapsulated lactate metal salt powder is cooled to room temperature under agitation.

5. Method according to any one of the preceding claims, wherein the powder of the lactate metal salt is ground to a smaller particle size prior to step (b).

6. Method according to any one of the preceding claims wherein the encapsulating agent is selected from hydrogenated oil, fat, wax, carbohydrates, proteins, polymers, and mixtures thereof.

7. Method according to any one of preceding claims wherein the weight ratio between the amount of encapsulating agent and the amount of lactate metal salt varies from 1:99 to 15:85, more preferably 4:96 to 8:92.

8. Method according to claim 1, wherein the concentrate of the lactate metal salt has been obtained by concentrating an aqueous solution of the alkali metal lactate.

9. Method according to any one of the preceding claims, wherein processing with cooling in a mixer/extruder is carried out at a starting temperature of 130° C to 170° C and cooling is carried out down to 10° C to 100° C.

10. Method according to claim 4, wherein the aqueous solution has been treated with active charcoal before it is concentrated.

11. Method according to any one of the preceding claims, wherein during processing with cooling in a mixer extruder the concentrate is cooled in a heat extractor column and then in a mixer/extruder.

12. Method according to any one of the preceding claims wherein the lactate metal salt is combined with a carrier.

13. Method according to claim 12, wherein the carrier is a flour, a starch, a silicate or an alkaline earth metal lactate.

14. Method according to claim 9 or 10 wherein the concentrate is combined with a carrier prior to being processed with cooling in a mixer/extruder to form a powder comprising the alkali metal lactate.

15. Method according to claim 9, wherein the lactate metal salt: carrier ratio ranges from 50 : 50 to 10:90, based on the weight of the lactate metal salt-comprising powder.

16. Method according to claim 12 wherein the carrier is mixed with the powder of the lactate metal salt prior to, during, or after the partial encapsulation of the powder of the lactate metal salt.

17. Method according to claim 13, wherein the lactate metal salt powder : carrier ratio ranges from 99 : 1 to 50:50, based on the weight of the lactate metal salt-comprising powder.

18. Partially encapsulated lactate metal salt powder obtainable by any of the methods of claims 1-14.

19. Partially encapsulated lactate metal salt powder which has a storage stability of at least one year.

20. Functional pre-mix comprising a partially encapsulated lactate metal salt powder according to claim 18 or 19.

21. Method for the preparation or preservation of foodstuff wherein a partially encapsulated lactate metal salt according to claim 18 or 19 is used.

22. Method for the preparation or preservation of foodstuff wherein a functional pre-mix according to claim 20 is used.

23. Foodstuff comprising a partially encapsulated lactate metal salt powder according to claim 18 or 19.

24. Foodstuff comprising a functional pre-mix according to claim 20.

25. Method for the preservation of cosmetic wherein a partially encapsulated lactate metal salt according to claim 18 or 19 is used.

26. Cosmetic comprising a partially encapsulated lactate metal salt according to claim 15 or 16.
